# EUROPEAN PATENT SPECIFICATION

(11) **EP 2 224 938 B1**
(45) Date of publication and mention of the grant of the patent: **27.07.2016**
(21) Application number: 08861477.1
(22) Date of filing: 02.12.2008
(51) Int. Cl.: A61K 36/67, A61P 35/00, A61P 35/02

(54) **EXTRACT OF PIPER BETEL LEAVES FOR THE TREATMENT OF HUMAN MALIGNANCIES BY INDUCING OXIDATIVE STRESS**
EXTRAKT VON PIPER BETEL BLÄTTERN ZUR BEHANDLUNG VON HUMANEN OXIDATIVEN STRESS INDUZIERENDEN KRANKHEITEN
EXTRAIT DE FEUILLES DE PIPER BETEL POUR LE TRAITEMENT DE TUMEURS MALIGNES HUMAINES PAR L'INDUCTION D'UN STRESS OXYDANT

(30) Priority: 17.12.2007 IN DE26402007
(43) Date of publication of application: 08.09.2010
(73) Proprietor: Council of Scientific & Industrial Research, New Delhi 110 001 (IN)
(72) Inventor: BANDYOPADHYAY, Santu, Kolkata (IN); PAL, Bikas, Chandra, Kolkata (IN); CHAKRABORTY, Jayashree, Bagchi, Kolkata (IN); RAKSHIT, Srabanti, Kolkata (IN); MANDAL, Labanya, Kolkata (IN); PAUL, Kausik, Kolkata (IN); BISWAS, Nabendu, Kolkata (IN); MANNA, Anirban, Kolkata (IN)
(74) Representative: Awapatent AB
(86) International application number: PCT/IN2008/000798
(87) International publication number: WO 2009/078035

(56) References cited:
- US-A1- 2003 229 140
- US-A1- 2005 089 585
- CHOUDHARY D ET AL: "Antioxidant and non-toxic properties of Piper betle leaf extract: in vitro and in vivo studies" MEDICINAL & AROMATIC PLANTS ABSTRACTS, RESOURCES, NEW DELHI, vol. 25, no. 4, 1 August 2003 (2003-08-01), XP018012677 ISSN: 0250-4367
- SARAVANAN R ET AL: "Influence of Piper betle on hepatic marker enzymes and tissue antioxidant status in Ethanol-treated Wistar rats." January 2002 (2002-01), JOURNAL OF MEDICINAL FOOD, VOL. 5, NR. 4, PAGE(S) 197-204 , XP009113055 ISSN: 1096-620X the whole document
- DATABASE WPI Week 200378 Thomson Scientific, London, GB; AN 2003-837160 XP002517563 & JP 2003 226649 A (DAIICHI PHARM CO LTD) 12 August 2003 (2003-08-12)
- NORTON ET AL: "Betel: Consumption and consequences" JOURNAL OF THE AMERICAN ACADEMY OF DERMATOLOGY, C.V. MOSBY, ST. LOUIS, MO, US, vol. 38, no. 1, 1 January 1998 (1998-01-01), pages 81-88, XP005686617 ISSN: 0190-9622
- RAO A R ET AL: "Inhibitory action of Piper betle on the initiation of 7,12-dimethylbenz[a]anthracene-induced mammary carcinogenesis in rats", CANCER LETTERS, NEW YORK, NY, US, vol. 26, no. 2, 1 March 1985 (1985-03-01), pages 207-214, XP026176517, ISSN: 0304-3835, DOI: 10.1016/0304-3835(85)90028-X [retrieved on 1985-03-01]

## Description

### FIELD OF THE INVENTION

This invention relates to extract of Piper betel leaves along with a pharmaceutically acceptable additive for use in the treatment of malignancies of different origin that are sensitive to oxidative stress in animals including human beings. Chemotherapy for cancer, in general, induce unacceptable toxicities. Broad spectrum anti-cancer activity of extract of *Piper betel* leaves without having significant toxicities in normal cells at the same dose range suggests its (extract of *Piper betel* leaves) potential use to treat human malignancies of different origin.

### BACKGROUND AND PRIOR ART OF THE INVENTION:

Reactive oxygen species (ROS) are natural byproducts of aerobic metabolism. ROS are produced in cells by a variety of enzymes and are detoxified by a series of proteins and small molecules. The various ROS can exert different effects according to their nature and to their intracellular level that are determined by both their production rate and the activity of antioxidant enzymes. Low level of ROS usually promotes cell proliferation while high concentration induces programmed cell death or apoptosis (Laurent A, Nicco C. et al. Cancer Res. 65: 948, 2005). Most cancerous cells exhibit elevated ROS generation compared to their normal counterparts (Burdon R.H. Free Radic Biol Med. 18: 775, 1995 ; Szatrowski T.P. and Nathan C.F. Cancer Res. 51: 794, 1991). High level of endogenous ROS in most cancerous cells provides an opportunity to exploit the potential of ROS to preferentially kill cancer cells over the normal ones by using ROS inducing agents. Elevated level of ROS in cancer cells make them highly vulnerable to ROS inducing agents as these agents increase the intracellular ROS in these cells to a toxic level or to the threshold that triggers cell death. On the other hand, normal cells can tolerate such an oxidative stress owing to their low endogenous ROS. Infact, ROS-mediated selective killing by phenylethyl isothiocyanate has been established for oncogenically transformed cancer cells (Trachootham D., Zhou Y. et al. Cancer Cell 10: 241, 2006). Redox manipulation of cancer cells has been emerging as an modern approach to treat varieties of cancers (Doroshow J.H. J. National Cancer Institute 98: 223, 2006).

Betel leaves have a strong pungent aromatic flavor and are widely used as a masticatory. Generally, mature or over mature leaves, which have ceased growing but not yet become brittle are used for chewing. The basic preparation for chewing purposes consists of betel leaf smeared with hydrated lime and catechu to which scrapings of arecanut are added; flavorings such as coconut shavings, clove, cardamom, fennel, powdered liquorice, nutmeg and also tobacco are used according to one's taste. In some places prepared pan is covered with silver or gold leaf. As a masticatory, it is credited with many properties: it is aromatic, digestive, stimulant and carminative. Medicinally, it is useful in catarrhal and pulmonary affections; it is also used for poultices. The effects of chewing of betel with arecanut and other adjuncts are the excitation of the salivary glands and the irritation of the mucous membrane of the mouth. The red coloration produced is due to a pigment in the arecanut, which manifests itself under the action of alkali in lime and catechu. A mild degree of stimulation is produced, resulting in a sensation of warmth and well being, besides imparting a pleasant odor. The most important factor determining the aromatic value of the leaf is the amount and particularly the nature of the essential oil present. Betel leaves from different regions vary in smell and taste. The most pungent is the Sanchi type, while the most mild and sweet ones are from Madras. The betel leaves contain essential oils, the content of oil varies from 0.7 to 2.6 per cent depending upon the varieties of leaves. The oil consists of phenols and terpens. The higher the proportion of phenol oil, the better the quality. An isomer of eugenol named chavibetol (betel phenol; 4-allyl-2-hydroxy-1-methoxy benzene) is considered to be the characteristic constituent of betel oil. It is however, absent in Indian samples. Betel oil of Indian types contain as a predominant phenolic constituent. Oil of betel has been used in the treatment of various respiratory catarrhs, as a local application either by gargle or by inhalation in diphtheria. It has carminative properties. It exhibits in different action on the central nervous system of mammals; lethal doses produce deep narcosis leading to death within a few hours. The essential oil and extracts of the leaves possess activity against several Gram-positive and Gram-negative bacteria such as Micrococcus pyogenes var. albus and var. aureus, Bacillus subtilis and B. megaterium, Diplococcus pneumoniae, Streptococcus pyogenes, Escherichia coli, Salmonella typhosa, Vibrio comma, Shigella dysenteriae, Proteus vulgaris, Pseudomonas solanacaerum, Sarcina lutea and Erwinia carotovora. The essential oil and leaf extracts also showed antifungal activity against Asperigillus niger and A. oryzae, Curvularia lunata and Fusarium oxysporum. The oil is found to be lethal in about 5 minutes to the protozoa Paramaecium caudatum (Wealth of India, Vol. 8, pg.84-94).

Chemoprevention of mammary tumor virus-induced and chemical carcinogen-induced rodent mammary tumors by *Piper betel* leaf extract has been published (Bhide VS., Azuinel MA. et al. J Breast Cancer Res and Treatment 30: 233, 1994; Rao AR., Sinha A. et al. Cancer Lett. 26: 207, 1985). Chemoprevention of cancer means inhibition of incidence of cancer development and is absolutely different than treatment of established cancers. Literature is also available on enhancement of sensitivity of hepatocarcinoma cells towards conventional chemotherapy by betel leaf extract (Young SC., Wang CJ. et al. Arch. Toxicol. 80: 319, 2006). However, therapeutic potential of extract of *Piper betel* leaf on cancers that are sensitive to oxidative stress is our novel finding. Anti-leishmanial activity (US Patent no. 6610332), immunomodulatory activity (US Patent no. 7045157), anti-acute and chronic myeloid leukemia activity of *Piper betel* leaf extract (A herbal composition for treating CD33 and chronic myeloid leukemia and a method thereof, US Patent application no. 20050089585; Series code: 10; Serial no.: 960064; Filed on October 08, 2004 ; antimonocytic activity of betel leaf extract, PCT application no. IN00/00118 and US Patent no. 6852344: A herbal composition for treating CD33⁺ acute and chronic myeloid leukemia and a method thereof) have been documented from our laboratory. Of note, prior arts US Patent no. 6852344 and PCT application no. IN00/00118 and US Patent application no. 20050089585 deal with treatment of acute and chronic myeloid leukemia only with *Piper betel* leaves extract. International patent publication (WO 2008/078203 A2) teaches the treatment of Gleevec-resistant chronic myeloid leukemia cells by *Piper betel* leaf extract by inhibition of Bcr-Abl kinase. On the other hand, the present invention deals with treatment of other cancers that are sensitive to oxidative stress. Recently, antioxidant (Choudhary D. and Kale R.K. Phytother Res. 16: 461, 2002; Santhakumri P, Prakasam A and Pugalendi KV. Indian Journal of Pharmacology, 2003, Vol. 35, 373-378) property of *Piper betel* leaves has been described. These prior arts suggested antioxidant activity of *Piper betel* i.e. attenuation of reactive oxygen species leading to protection against tissue lipid peroxidation in diabetic rats. In contrast, our present patent application presented evidence indicating that *Piper betel* leaf extract induces oxidative stress leading to preferential killing of cancer cells over normal cells. This is explained as follows: In general, cancer cells have higher level of intracellular reactive oxygen species, close to the threshold of cytotoxicity, compared to normal cells. Therefore, any further increase in intracellular reactive oxygen species in cancer cells leads to toxicity. On the other hand, since the basal level of reactive oxygen species in normal cells is low, further increase upto certain point is not associated with normal cell death. Radioprotective (Bhattacharya S., Subramanian M. et al. J. Radiat. Res. 46: 165, 2005) and anti-inflammatory properties (Ganguly S., Mula S. et al. J. Pharmacy and Pharmacology 59: 711, 2007) of *Piper betel* leaf extract have also been published.

| **Prior art** | **Cell / Cell lines** | **Type of cancer** | **Application No.** | **Applicant** |
|---|---|---|---|---|
| A herbal composition for treating CD33+ acute and chronic myeloid leukemia and a method thereof. | Myeloid leukemia cells | Acute & chronic myeloid leukemia | US20070730433, US20040960064, US20030448398, US20020207039 | Council of Scientific & Industrial Research |
| | Ba/F3 Bcr-Abl P210 WT | | | |
| | Ba/F3 Bcr-Abl T315I | | | |
| | Ba/F3 Bcr-Abl E255K | | | |
| | Ba/F3 Bcr-Abl H396P | | | |
| A herbal composition and process for its preparation. | Ba/F3 Bcr-Abl M351T | Chronic myeloid leukemia, resistance with imatinab (gleevec or glivec) | PCT/IB2007/050536 | Piramal Life Sciences Limited |
| | Ba/F3 B3cr-Abl F359V | | | |
| | K-562 | | | |
| | Ba/F3 Bcr-Abl P210 | | | |
| | Ba/F3 Bcr-Abl E255V | | | |
| | Ba/F3 Bcr-Abl F317V | | | |
| | Ba/F3 Bcr-Abl H396R | | | |
| | Ba/F3 Bcr-Abl M284V | | | |
| | Ba/F3 Bcr-Abl Q252H | | | |
| | Ba/F3 Bcr-Abl Y253F | | | |
| | Ba/F3 Bcr-Abl Y253H | | | |
| | HL-60 | | | |
| | Molt-4 | | | |

| **Present invention** | **Cell / Cell lines** | **Type of cancer** | **Application No.** | **Applicant** |
|---|---|---|---|---|
| Extract of *Piper betel* leaves for the treatment of human malignancies by inducing oxidative stress | HepG-2 | Hepatocellular carcinoma (for comparison only) | 2640DEL2007 | Council of Scientific & Industrial Research |
| | HSG | Salivary gland carcinoma (for comparison onlv) | | |
| | MIA PaCa-2 | Pancreatic carcinoma | | |
| | PC-3 | Prostate carcinoma | | |
| | MCF-7 | Breast adenocarcinoma (for comparison only) | | |
| | HeLa | Cervix carcinoma | | |
| | A-375 | Skin melanoma | | |
| | Daudi | Human burkitt lymphoma | | |
| | EAC | Ehrlich ascites carcinoma | | |
| | REH | B-cell precursor leukemia (for comparison only) | | |

Therefore, none of the prior arts on *Piper betel* leaf extract relate to the present application which deals with broad spectrum anti-cancer activity of *Piper betel* leaf extract by inducing oxidative stress (reactive oxygen species).

### OBJECTS OF THE INVENTION

The main object of the invention is to provide a method of treating human beings for treating malignancies of different origin which are sensitive to oxidative stress by the way of administering extract of *Piper betel* leaves along with a pharmaceutically acceptable additives.

Another object of the invention is to provide a method for treating human malignancies which are sensitive to oxidative stress that include pancreatic carcinoma, prostrate carcinoma, cervix carcinoma, skin melanoma, human burkitt lymphoma, ehrlich ascites carcinoma with extract of *Piper betel* leaves.

Another object of the present invention is to provide a composition comprising extract of *Piper betel* leaves along with a pharmaceutically acceptable additives which is useful for the treatment of human malignancies which are sensitive to oxidative stress that include pancreatic carcinoma, prostrate carcinoma, cervix carcinoma, skin melanoma, human burkitt lymphoma, ehrlich ascites carcinoma.

Another objective of the invention is to provide a method of treating said human malignancies by inducing oxidative stress with the extract of *Piper betel* leaves along with pharmaceutically acceptable additives.

Yet another objective of the invention is to provide a composition comprising extract of *Piper betel* leaves along with pharmaceutically acceptable additives for oxidative stress-induced killing of said human malignancies.

### SUMMARY OF THE INVENTION:

Accordingly, the present invention provides use of extract of *Piper betel* leaves for the treatment of human malignancies of different origin by inducing oxidative stress.

### BRIEF DESCRIPTION OF THE ACCOMPANYING DRAWINGS:

**Fig. 1****:** Extract of *Piper betel* leaves is effective *in vivo* in destroying Ehrilich ascites carcinoma.
**Fig. 2**: Extract of *Piper betel* leaves induces oxidative stress (H₂O₂) in EAC tumor cells but not in normal human peripheral blood mononuclear cells (PBMC): oxidative stress induced by extract of *Piper betel* leaves is scavenged by treatment with Catalase.
**Fig. 3****:** Extract of *Piper betel* leaves induces apoptosis and necrosis in EAC tumor cells but not in normal human peripheral blood mononuclear cells (PBMC): Extract of *Piper betel* leaves-induced death is reversed by Catalase treatment.
**Fig. 4****:** Extract of *Piper betel* leaves inhibits growth of human breast adenocarcinoma grafted in nude mice.

### DETAILED DESCRIPTION OF THE INVENTION:

The present invention provides composition comprising extract of *Piper betel* leaves along with pharmaceutically acceptable additives useful for treating human malignancies of different origin by inducing oxidative stress. The malignancies include but not limited to pancreatic carcinoma, prostrate carcinoma, cervix carcinoma, skin melanoma, human burkitt lymphoma, ehrlich ascites carcinoma.
In yet another embodiment the present invention provides a method of treating said human malignancies by using extract of *Piper betel* leaves along with pharmaceutically acceptable additives.

In yet another embodiment of the present invention wherein, the additive may be selected from a group consisting of nutrients such as proteins, carbohydrates, sugars, talc, magnesium stearate, cellulose, calcium carbonate, starch-gelatin paste and or pharmaceutically acceptable carriers, excipients, diluents or solvents.

In yet another embodiment of the present invention, wherein the said composition is formulated for oral administration in the form of a tablet, capsule, granules, syrup or suspension.

It is yet another embodiment of the present invention to use the extract of *Piper betel* leaves for the treatment of human malignancies which are sensitive to oxidative stress that include pancreatic carcinoma, prostrate carcinoma, cervix carcinoma, skin melanoma, human burkitt lymphoma, ehrlich ascites carcinoma.

It is yet another embodiment of the present invention to use the extract of *Piper betel* leaves, wherein minimum concentration to induce 50% cell death (IC50) ranging between 5µg/ml-10µg/ml.

It is further another embodiment of the present invention to use the extract of of *Piper betel* leaves, wherein the said extract is administered through oral, intravenous, intramuscular or subcutaneous routes in an individual suffering from malignancies which are sensitive to oxidative stress that include pancreatic carcinoma, prostrate carcinoma, cervix carcinoma, skin melanoma, human burkitt lymphoma, ehrlich ascites carcinoma.

It is yet another embodiment of the present invention to use the extract of *Piper betel* leaves, wherein the extract is administered in a concentration ranging between 250-1000mg/kg of body weight.

It is yet another embodiment of the present invention to use the extract of *Piper betel* leaves, wherein the extract administered orally destroys the ehrlich ascites tumor cells at a dose ranging between 250-500mg/kg of body weight.

It is yet another embodiment of the present invention to use the extract of *Piper betel* leaves, wherein the extract enhances intracellular H₂O₂ concentration in ehrlich ascites carcinoma cells (EAC) but not in normal human peripheral blood mononuclear cells(PBMC).

It is yet another embodiment of the present invention to use the extract of *Piper betel* leaves, wherein the extract induces both apoptosis and necrosis in ehrlich ascites carcinoma cells (EAC) but not in normal human peripheral blood mononuclear cells (PBMC).

It is yet another embodiment of the present invention to use the extract of of *Piper betel* leaves, wherein the extract induces both apoptosis and necrosis in cancer cells that include pancreatic carcinoma, prostrate carcinoma, cervix carcinoma, skin melanoma, human burkitt lymphoma, ehrlich ascites carcinoma.

In yet another embodiment of the present invention wherein, the said composition is administered through oral, intravenous, intramuscular or subcutaneous routes in an individual suffering from said malignancies.

In yet another embodiment, the said composition is administered in a concentration of 250 mg/kg of said individual's body weight to about 1000 mg/kg of said individual's body weight.

In yet another embodiment, the said composition preferentially induces oxidative stress in cancer cells of different origin.

In yet another embodiment, the said composition induces apoptosis and necrosis preferentially in cancer cells.

In yet another embodiment, the said composition induces apoptosis and necrosis of Ehrlich ascites tumor cells *in vitro.*

In yet another embodiment, the said composition destroys Ehrlich ascites tumor cells *in vivol* in mouse model.

In yet another embodiment, the said composition destroys pancreatic carcinoma (MIA PaCa-2) *in vitro.*

In yet another embodiment, the said composition destroys prostrate carcinoma (PC-3) *in vitro.*

In yet another embodiment, the said composition destroys cervix carcinoma (HeLa) *in vitro.*

In yet another embodiment, the said composition destroys skin melanoma (A-375) *in vitro.*

In yet another embodiment, the said composition destroys human burkitt lymphoma (Daudi) *in vitro.*

In yet another embodiment, the said composition does not affect the viability of normal human peripheral blood mononuclear cells (PBMC) at the same dose range *in vitro.*

It is expected that the composition comprising extract of *Piper betel* leaves along with pharmaceutically acceptable additives would be useful in an individual suffering from one of the malignancies that are sensitive to oxidative selected from pancreatic carcinoma, prostrate carcinoma, cervix carcinoma, skin melanoma, human burkitt lymphoma, ehrlich ascites carcinoma.

**The invention is described in detail with reference to the examples given below which are provided to illustrate the invention.**

### Example 1: Collection of plant +material

The leaves of *Piper betel* were collected from different areas of West Bengal, India. A voucher specimen was deposited at the Department of Medicinal Chemistry at the Indian Institute of Chemical Biology, 4 Raja S.C. Mullick Road, Kolkata-700 032.

### Example 2: Preparation of extract of Piper betel leaves (PB Extract)

5.0 kg fresh leaves of *Piper betel* is made a paste in a mixture-blender with an organic solvent (2 lit.) like alcohols, esters, ethers, petroleum ether, ketones, hexane, chloroform and/or water and is placed in a glass percolator (5 lit capacity) with the addition of 1000 ml of the solvent. It was allowed to extract for 16 hrs (overnight). The extract was filtered through Whatman No.1 filter paper and the filtrate was collected. This process of extraction was repeated for three times (3lit.x3). The combined extract was evaporated to dryness in flash evaporator under reduced pressure to remove the solvent completely. The residual substance was then dried in a desiccator under high vacuum and the semi-solid mass weighing 106.5 gm was tested for biological activity.

### Properties of the Materials:

The biologically active material obtained by example 2 has the following properties:
**I.** The dried semi-solid prepared as stated above was a dark colored material soluble in ethanol/methanol/dimethylsulfoxide.
**II.** Thin layer chromatography of the active material shows six spots having R_{f} 0.84, 0.58, 0.51, 0.45, 0.40 and 0.34 in the solvent system of n-butanol, acetic acid and water in the ratio of 13:3:5 respectively. The spots are observed in UV chamber at wavelength 254 nm.
**III.** The HPLC analysis of the active material using C₁₈ Xterra (Waters) (4.6 x 250 mm) analytical column, solvent system using water: methanol: acetic acid (76:23:1) and a flow rate of 1 ml/min., detection in UV at 210 nm resolved the material into nine peaks with the retention time of 5.71, 6.53, 8.01, 12.02, 15.27, 16.91, 23.15, 28.24 and 45.70 min.

### Example 3: Effects of extract of Piper betel leaves (PB Extract) on viability of cancer cell lines of different origin and normal human peripheral blood mononuclear cells (PBMC) in vitro

Following cancer cell lines were used: Prostate cancer cell line PC-3; breast cancer cell line MCF-7 (for comparison only); pancreatic cancer cell line MIA PaCa-2; cervix carcinoma HeLa; skin melanoma A-375; human burkitt lymphoma Daudi; B-cell precursor leukemia REH (for comparison only). These cancer cell lines were maintained in tissue culture in standard growth medium containing 10% heat-inactivated fetal bovine serum (FBS).

Ehrlich ascites carcinoma (EAC) was established in Swiss albino mice as described (Pal S. et al. B.B.RC. 288: 658-665, 2001). Swiss albino mice were injected with EAC (2 □ 10⁶ cells/mouse) intraperitoneally. Three weeks later, EAC cells were removed from peritoneal cavity for culture.

Normal human peripheral blood mononuclear cells (PBMC) were separated from whole blood by Ficoll/Hypaque density gradient centrifugation. Peripheral blood samples were collected from normal donors with due approval from the Human Ethics Committee of Indian Institute of Chemical Biology and all experiments with human blood were conducted under an approved institutional Human Ethics committee protocol. Informed consent was provided according to the Declaration of Helsinki. Cancer cell lines, EAC cells isolated from peritoneal cavity of Swiss albino mice and PBMC (5 □ 10³) in triplicate were incubated in 0.2 ml of standard growth medium containing 10% FBS with varying concentrations of extract of *Piper betel* leaves. After 72 hours of incubation, cells were collected by centrifugation (at 1000 g for 5 minutes) and cell-viability was determined by the trypan blue exclusion assay. At least 200 cells were examined in each sample. Monolayer cultures were detached from the wells by treatment with Cell Dissociation Solution (Sigma Chemical, St. Louis, MO) before counting. Data are represented as IC₅₀ (minimum concentration required to inhibit the viability by 50%).

To evaluate whether the anti-cancer activity of the extract of *Piper betel* leaves is specific or the activity is present in any plant extract, extract of *Leucas linifolia* was also prepared and evaluated against the indicated cell lines. This extract was used as a control.

*Leucas linifolia* is a herbaceous annual weed which grows abundantly in fields, pastures and waste lands throughout India. This herb is reported to have sedative, vermifuge, stomachic and dermatosis activity [Sastri, B.N. (ed) (1962). The Wealth of India, Raw Materials, Vol. VI. P80. CSIR, New Delhi].

### Example 4: In vivo efficacy of extract of Piper betel leaves (PB Extract) on Ehrlich ascites carcinoma (EAC) model

Swiss albino mice were injected with EAC (2 □ 10⁶ cells/mouse) intraperitoneally. 2 days post-injection of tumor cells, these mice (5 per group) were fed orally extract of *Piper betel* leaves. (250 mg/kg body weight; 500 mg/kg body weight) thrice a week for three weeks. One week after the last injection, mice were sacrificed, and viable cells from peritoneal cavity were counted microscopically. Vehicle alone (0.2 ml of 0.2% ethanol per feeding) was used as control.

### Example 5: Extract of Piper betel leaves induces oxidative stress (H₂O₂) preferably in cancer cells

Ehrlich ascites carcinoma (EAC) cells isolated from peritoneal fluid of Swiss albino mice and normal human peripheral blood mononuclear cells (PBMC) were incubated with extract of *Piper betel* leaves (10 □ g/ml) for absence of Catalase (2000 U/ml, Calbiochem, San Diago, USA; Catalase is an enzyme which specifically destroys H₂O₂ by converting it to H₂O and O₂). Cells were washed and treated with dichlorofluorescein diacetate (DCF, from Calbiochem) at a concentration of 10 □ M for 15 min a BD LSR flow cytometer (Becton Dickinson, San Jose, CA). Data are presented as histograms. DCF reportedly detects intracellular H₂O₂ (Chandra J. et al. Blood 102: 4512, 2003). Values in the histograms represent mean fluorescence intensity.

### Example 6: Extract of Piper betel leaves induces apoptosis and necrosis preferentially in cancer cells: PB-extract-induced apoptosis and necrosis in cancer cells is induced by oxidative stress and reversed by Catalase treatment

EAC cells and PBMC were treated with vehicle control (0.2% ethanol v/v) or extract of *Piper betel* leaves (10 □ g/ml) f Catalase (2000 U/ml). After washing, cells were stained with fluorescein isothiocyanate (FITC)-conjugated annexin V and propidium iodide (PI) and analysed in a flow cytometer (BD LSR, Becton Dickinson). Data are presented as dot plots. Early apoptotic cells stained by annexin-V are in the lower-right quadrant. Late-stage apoptotic cells stained with both annexin-V and PI are in the upper-right quadrant. Cells present in the upper-left quadrant are necrotic cells stained only by PI. Cells present in lower-left quadrant are unstained by both annexin-V and PI and are live cells (Bandyopadhyay G., Biswas T. et al. Blood 104:2514, 2004). Values in each quadrant represent percent positive cells.

### Example 7: Extract of Piper betel leaves inhibits growth of human breast adenocarcinoma (MCF-7) grafted in nude mice (for comparison only)

MCF-7 cells were suspended to 5×10⁷ cells/ml in Matrigel (BD Biosciences; 1 volume of cells with 1 volume of cold Matrigel). Nude female mice of 6 to 7 weeks of age (National Institute of Nutrition, Hyderabad, India) were injected with 0.2 ml of this cell suspension. Animals were left untreated until MCF-7 xenografts reached 200-300 mm³. PB extract of varying doses (500-1000 mg/kg) or vehicle control (0.2 ml of 0.2% ethanol per feeding) were administered via oral route twice a day for 10 days (5 mice per group)

### Observed Results:

### Results of Example 3:

Extract of *Piper betel* leaves induced killing of cancer cells lines of different origin i.e. pancreatic carcinoma, prostrate carcinoma, cervix carcinoma, skin melanoma, human burkitt lymphoma, Ehrilich ascites carcinoma (Table 1). Normal human peripheral blood mononuclear cells (PBMC) requires much higher dose of the extract to observe cytotoxicity (Table 1), suggesting a wide therapeutic window for the possible treatment of these cancers with this extract.

In contrast, extract of *Leucas linifolia* has very weak activity against the cancer cell lines (Table 2) compared to *Piper betel* leaf extract.

### Results of Example 4:

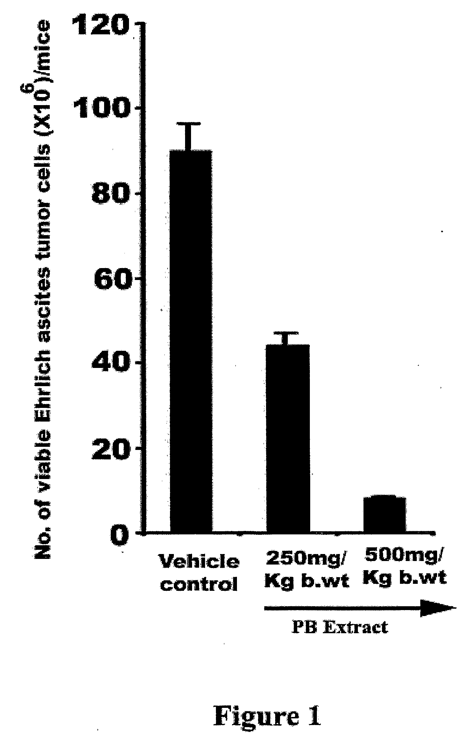

Extract of *Piper betel* leaves when administered orally in Swiss albino mice destroyed EAC tumor cells in a dose dependent manner (Fig. 1). These data indicate that the extract *of Piper betel* leaves is effective *in vivo* in destroying cancer cells.

### Results of Example 5:

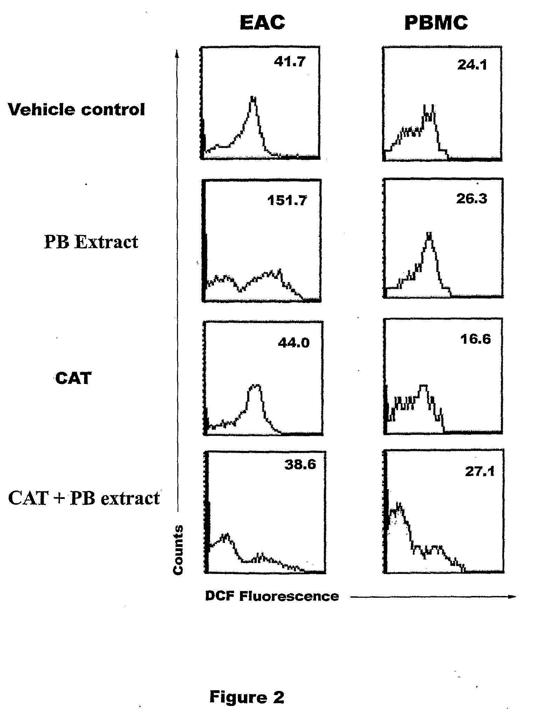

Treatment with extract of *Piper betel* leaves enhances intracellular H₂O₂ concentration in EAC tumor cells but not in normal human peripheral blood mononuclear cells (PBMC) as determined by Flow cytometry-based studies after staining with DCF (Fig. 2). Catalase treatment reverses the effect, confirming the enhancement of intracellular H₂O₂ in EAC cells by extract of *Piper betel* leaves (Fig. 2).

### Results of Example 6:

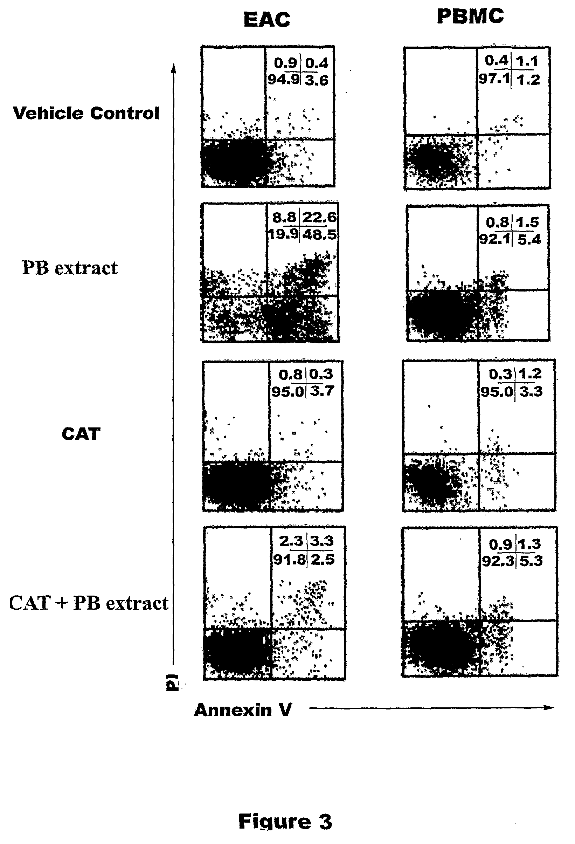

Flow cytometry-based studies after staining with annexin-V FITC/PI indicate that extract of *Piper betel* leaves induces both apoptosis and necrosis in EAC tumor cells but not in normal human peripheral blood mononuclear cells (PBMC) (Fig. 3). Our data also indicate that extract of *Piper betel* leaves-induced death of cancer cells is solely mediated by oxidative stress as Catalase treatment reverses the effect (Fig. 3).

### Results of Example 7:

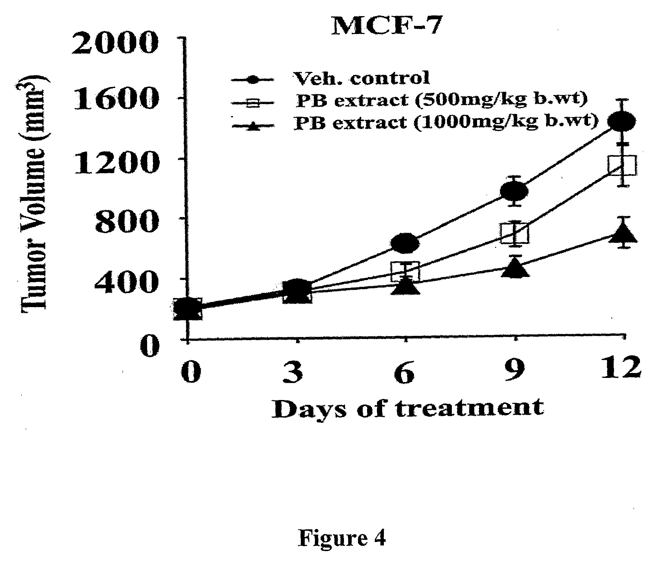

The *in vivo* efficacy of *Piper betel* leaves extract for anti-cancer activity was further confirmed in human adenocarcinoma (MCF-7) xenografts in nude mice (Fig.4).

**Table 1: In vitro activity of extract of Piper betel leaves against cancer cell lines of different origin and normal human peripheral blood mononuclear cells (PBMC)**

| **Cell type** | **Activity (IC₅₀[□ g/ml])** |
|---|---|
| **MIA PaCa-2 (Pancreatic carcinoma)** | 10.0 |
| **PC-3 (Prostate carcinoma)** | 7.0 |
| **MCF-7 (Breast adenocarcinoma) (for comparison only)** | 7.0 |
| **HeLa (Cervix carcinoma)** | 7.0 |
| **A-375 (Skin melanoma)** | 7.0 |
| **Daudi (Human burkitt lymphoma)** | 5.0 |
| **EAC (Ehrlich ascites carcinoma)** | 5.0 |
| **REH (B-cell precursor leukemia) (for comparison only)** | 5.0 |
| **PBMC (Normal human peripheral blood mononuclear cells)** | 30.0 |

| | |
|---|---|
| *Cell count assays were performed by plating cells in regular growth medium in the absence and presence of varying concentrations of extract of *Piper betel* leaves. Viable cells were counted as assessed by exclusion of trypan blue. Data are presented as IC₅₀ (minimum concentration needed to induce 50% death). | |

**Table 2: In vitro activity of extract of Leucas linifolia against cancer cell lines of different origin and normal human peripheral blood mononuclear cells (PBMC)**

| **Cell type** | **Activity (IC₅₀)** |
|---|---|
| **MIA PaCa-2 (Pancreatic carcinoma)** | 95.0 |
| **PC-3 (Prostate carcinoma)** | 93.0 |
| **MCF-7 (Breast adenocarcinoma) (for comparison only)** | 95.0 |
| **HeLa (Cervix carcinoma)** | 89.0 |
| **A-375 (Skin melanoma)** | Not done |
| **Daudi (Human burkitt lymphoma)** | 89.7 |
| **EAC (Ehrlich ascites carcinoma)** | 97.3 |
| **REH (B-cell precursor leukemia) (for comparison only)** | 95.4 |
| **PBMC (Normal human peripheral blood mononuclear cells)** | 100.4 |

| | |
|---|---|
| *Cell count assays were performed by plating cells in regular growth medium in the absence and presence of varying concentrations of extract of *Leucas linifolia.* Viable cells were counted as assessed by exclusion of trypan blue. Data are presented as IC₅₀ (minimum concentration needed to induce 50% death). | |

## Claims

1. A composition comprising extract of *Piper betel* leaves along with a pharmaceutically acceptable additive for use in the treatment of human malignancies which are sensitive to oxidative stress selected from a group consisting of pancreatic carcinoma, prostrate carcinoma, cervix carcinoma, skin melanoma, human burkitt lymphoma and ehrlich ascites carcinoma.

2. A composition as claimed in claim 1, wherein the pharmaceutically acceptable additive can be selected from a group consisting of nutrients such as proteins, carbohydrates, sugars; talc, magnesium stearate, cellulose, calcium carbonate, starch-gelatin paste and/or pharmaceutically acceptable carriers, excipients, diluents or solvents.

3. A composition as claimed in claim 1, wherein the said composition is formulated for oral administration in the form of a tablet, capsule, granules, syrup or suspension.

4. Use of extract of *Piper betel* leaves for the manufacture of a medicament for the treatment of human malignancies which are sensitive to oxidative stress selected from a group consisting of pancreatic carcinoma, prostrate carcinoma, cervix carcinoma, skin melanoma, human burkitt lymphoma and ehrlich ascites carcinoma.

5. Use of extract of *Piper betel* leaves as claimed in claim 4, wherein minimum concentration to induce 50% cell death (IC50) ranging between 5 µg/ml- 10µg/ml.

6. Use of extract of *Piper betel* leaves as claimed in claim 4, wherein the said extract is administrable through oral, intravenous, intramuscular or subcutaneous routes in an individual suffering from a malignancy which is sensitive to oxidative stress selected from a group consisting of pancreatic carcinoma, prostrate carcinoma, cervix carcinoma, skin melanoma, human burkitt lymphoma and ehrlich ascites carcinoma.

7. Use of extract of *Piper betel* leaves as claimed in claim 4, wherein the extract is administered in a concentration ranging between 250-1000 mg/kg of body weight.

8. Use of extract of *Piper betel* leaves as claimed in claim 4, wherein the extract administered orally destroys the ehrlich ascites tumor cells at a dose ranging between 250-500 mg/kg of body weight.

9. Use of extract of *Piper betel* leaves as claimed in claim 4, wherein the extract enhances intracellular H₂O₂ concentration in ehrlich ascites carcinoma cells (EAC) but not in normal human peripheral blood mononuclear cells (PBMC).

10. Use of extract of *Piper betel* leaves as claimed in claim 4, wherein the extract induces both apoptosis and necrosis in ehrlich ascites carcinoma cells (EAC) but not in normal human peripheral blood mononuclear cells (PBMC).

11. Use of extract of *Piper betel* leaves as claimed in claim 4, wherein the extract induces both apoptosis and necrosis in cancer cells selected from a group consisting of pancreatic carcinoma, prostrate carcinoma, cervix carcinoma, skin melanoma, human burkitt lymphoma and ehrlich ascites carcinoma.

## Patentansprüche

1. Zusammensetzung, umfassend Extrakt von *Piper-betel*-Blättern zusammen mit einem pharmazeutisch unbedenklichen Zusatzstoff zur Verwendung in der Behandlung von humanen Malignitäten, die gegenüber Oxidationsstress empfindlich sind, ausgewählt aus einer Gruppe bestehend aus Pankreaskarzinom, Prostatakarzinom, Cervixkarzinom, Hautmelanom, Burkitt-Lymphom des Menschen und Ehrlich-Asciteskarzinom.

2. Zusammensetzung nach Anspruch 1, wobei der pharmazeutisch unbedenkliche Zusatzstoff aus einer Gruppe bestehend aus Nährstoffen wie Proteinen, Kohlenhydraten, Zuckern; Talk, Magnesiumstearat, Zellulose, Calciumcarbonat, Stärke-Gelatine-Paste und/oder pharmazeutisch unbedenklichen Trägern, Grundstoffen, Verbindungsmitteln oder Lösungsmitteln ausgewählt werden kann.

3. Zusammensetzung nach Anspruch 1, wobei die Zusammensetzung für die orale Verabreichung in Form einer Tablette, einer Kapsel, eines Granulats, eines Sirups oder einer Suspension formuliert ist.

4. Verwendung von Extrakt von *Piper-betel-*Blättern für die Herstellung eines Arzneimittels für die Behandlung von humanen Malignitäten, die gegenüber Oxidationsstress empfindlich sind, ausgewählt aus einer Gruppe bestehend aus Pankreaskarzinom, Prostatakarzinom, Cervixkarzinom, Hautmelanom, Burkitt-Lymphom des Menschen und Ehrlich-Asciteskarzinom.

5. Verwendung von Extrakt von *Piper-betel-*Blättern nach Anspruch 4, wobei die Minimalkonzentration, um 50% Zelltod zu induzieren (IC50) im Bereich zwischen 5 µg/ml - 10 µg/ml liegt.

6. Verwendung von Extrakt von *Piper-betel-*Blättern nach Anspruch 4, wobei der Extrakt über orale, intravenöse, intramuskuläre oder subkutane Wege an ein Individuum verabreichbar ist, das an einer Malignität, die gegenüber Oxidationsstress empfindlich ist, leidet, ausgewählt aus einer Gruppe bestehend aus Pankreaskarzinom, Prostatakarzinom, Cervixkarzinom, Hautmelanom, Burkitt-Lymphom des Menschen und Ehrlich-Asciteskarzinom.

7. Verwendung von Extrakt von *Piper-betel-*Blättern nach Anspruch 4, wobei der Extrakt in einer Konzentration im Bereich zwischen 250-1000 mg/kg Körpergewicht verabreicht wird.

8. Verwendung von Extrakt von *Piper-betel*-Blättern nach Anspruch 4, wobei der oral verabreichte Extrakt die Ehrlich-Ascitestumorzellen in einer Dosierung im Bereich von 250-500 mg/kg Körpergewicht zerstört.

9. Verwendung von Extrakt von *Piper-betel*-Blättern nach Anspruch 4, wobei der Exrakt die intrazelluläre H₂O₂-Konzentration in Ehrlich-Asciteskarzinomzellen (EAC), jedoch nicht in normalen menschlichen mononukleären Zellen des peripheren Bluts (PBMC), erhöht.

10. Verwendung von Extrakt von *Piper-betel-*Blättern nach Anspruch 4, wobei der Extrakt sowohl Apoptose als auch Nekrose in Ehrlich-Asciteskarzinomzellen(EAC), jedoch nicht in normalen menschlichen mononukleären Zellen des peripheren Bluts (PBMC) induziert.

11. Verwendung von Extrakt von *Piper-betel-*Blättern nach Anspruch 4, wobei der Extrakt sowohl Apoptose als auch Nekrose in Krebszellen, ausgewählt aus einer Gruppe bestehend aus Pankreaskarzinom, Prostatakarzinom, Cervixkarzinom, Hautmelanom, Burkitt-Lymphom des Menschen und Ehrlich-Asciteskarzinom, induziert.

## Revendications

1. Composition comprenant un extrait de feuilles de *Piper betle* ainsi qu'un adjuvant pharmaceutiquement acceptable pour utilisation dans le traitement de tumeurs malignes humaines sensibles au stress oxydant choisies dans un groupe constitué du carcinome pancréatique, du carcinome prostatique, du carcinome du col de l'utérus, des mélanomes cutanés, du lymphome de Burkitt humain et du carcinome à cellules ascitiques d'Ehrlich.

2. Composition selon la revendication 1, où l'adjuvant pharmaceutiquement acceptable peut être choisi dans un groupe constitué par les nutriments tels que protéines, hydrates de carbone, sucres ; le talc, le stéarate de magnésium, la cellulose, le carbonate de calcium, la pâte d'amidon-gélatine et/ou les vecteurs, excipients, diluants ou solvants pharmaceutiquement acceptables.

3. Composition selon la revendication 1, où ladite composition est formulée pour administration orale sous forme d'un comprimé, d'une capsule, de granules, d'un sirop ou d'une suspension.

4. Utilisation d'un extrait de feuilles de *Piper betle* dans la fabrication d'un médicament destiné au traitement de tumeurs malignes humaines sensibles au stress oxydant choisies dans un groupe constitué du carcinome pancréatique, du carcinome prostatique, du carcinome du col de l'utérus, des mélanomes cutanés, du lymphome de Burkitt humain et du carcinome à cellules ascitiques d'Ehrlich.

5. Utilisation d'un extrait de feuilles de *Piper betle* selon la revendication 4, où la concentration minimale permettant d'induire 50 % de mort cellulaire (CI₅₀) est comprise entre 5 µg/ml et 10 µg/ml.

6. Utilisation d'un extrait de feuilles de *Piper betle* selon la revendication 4, où ledit extrait est administrable par voie orale, intraveineuse, intramusculaire ou sous-cutanée chez un individu souffrant d'une tumeur maligne sensible au stress oxydant choisie dans un groupe constitué du carcinome pancréatique, du carcinome prostatique, du carcinome du col de l'utérus, des mélanomes cutanés, du lymphome de Burkitt humain et du carcinome à cellules ascitiques d'Ehrlich.

7. Utilisation d'un extrait de feuilles de *Piper betle* selon la revendication 4, où l'extrait est administré à une concentration comprise entre 250 et 1000 mg/kg de poids corporel.

8. Utilisation d'un extrait de feuilles de *Piper betle* selon la revendication 4, où l'extrait administré par voie orale détruit les cellules tumorales ascitiques d'Ehrlich à une dose comprise entre 250 et 500 mg/kg de poids corporel.

9. Utilisation d'un extrait de feuilles de *Piper betle* selon la revendication 4, où l'extrait amplifie la concentration intracellulaire en H₂O₂ dans les cellules ascitiques d'Ehrlich (EAC), mais pas dans les cellules mononucléaires de sang périphérique (PBMC) humain normales.

10. Utilisation d'un extrait de feuilles de *Piper betle* selon la revendication 4, où l'extrait induit à la fois l'apoptose et la nécrose dans les cellules ascitiques d'Ehrlich (EAC), mais pas dans les cellules mononucléaires de sang périphérique (PBMC) humain normales.

11. Utilisation d'un extrait de feuilles de *Piper betle* selon la revendication 4, où l'extrait induit à la fois l'apoptose et la nécrose dans les cellules cancéreuses choisies dans un groupe constitué du carcinome pancréatique, du carcinome prostatique, du carcinome du col de l'utérus, des mélanomes cutanés, du lymphome de Burkitt humain et du carcinome à cellules ascitiques d'Ehrlich.
